(19) Europäisches Patentamt
European Patent Office
Office européen des brevets

(11) Numéro de publication: **0 149 407**
**B1**

(12) # FASCICULE DE BREVET EUROPEEN

(45) Date de publication du fascicule du brevet: **01.04.87**

(51) Int. Cl.⁴: **C 07 C 45/63**, C 07 C 47/575, C 07 C 47/565

(21) Numéro de dépôt: **84420211.9**

(22) Date de dépôt: **19.12.84**

(54) **Procédé de préparation de bromobenzaldehydes hydroxy et/ou alkoxy substitués.**

(30) Priorité: **22.12.83 FR 8320797**

(43) Date de publication de la demande: **24.07.85 Bulletin 85/30**

(45) Mention de la délivrance du brevet: **01.04.87 Bulletin 87/14**

(84) Etats contractants désignés: **AT BE CH DE GB IT LI LU NL SE**

(56) Documents cités:
**FR - A - 2 274 586**
**US - A - 3 303 224**

**Le dossier contient des informations techniques présentées postérieurement au dépôt de la demande et ne figurant pas dans le présent fascicule.**

(73) Titulaire: **RHONE-POULENC SPECIALITES CHIMIQUES, "Les Miroirs" 18, Avenue d'Alsace, F-92400 Courbevoie (FR)**

(72) Inventeur: **Ratton, Serge, Hameau de Belmont Vaulx-Milieu, F-38090 - Villefontaine (FR)**
Inventeur: **Bougeois, Jean-Luc, 4, Chemin du Signal, F-69110 Sainte-Foy-ls-Lyon (FR)**

(74) Mandataire: **Vignally, Noel et al, RHONE-POULENC INTERSERVICES Service Brevets Chimie Centre de Recherches de Saint-Fons B.P. 62, F-69192 Saint-Fons Cedex (FR)**

**EP 0 149 407 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation de bromobenzaldéhydes comportant des substituants hydroxy et/ou alkoxy et plus particulièrement de la bromo-5 vanilline.

Les bromobenzaldéhydes comportant des substituants hydroxy et/ou alkoxy sont des produits industriels précieux utilisés comme intermédiaire en synthèse organique. Ainsi la bromo-5 vanilline (bromo-3 hydroxy-4 méthoxy-5 benzaldéhyde), l'aldéhyde bromoprotocatéchique (bromo-3 dihydroxy-4,5 benzaldéhyde) et le bromo-3 diméthoxy-4,5 benzaldéhyde sont utilisés comme intermédiaires pour la préparation du triméthoxy-3,4,5 benzaldéhyde qui intervient lui-même dans la fabrication de produits pharmaceutiques tels que le triméthoprim [diamino -2,4 (triméthoxy-3,4,5 benzyl)-5 pyrimidine]. Ces bromobenzaldéhydes sont également utilisés pour la préparation de bromophénylalanines possédant une activité comme hypotenseur (cf. brevet français 1 592 518).

Les bromobenzaldéhydes alkoxy et/ou hydroxy substitués sont préparés par réaction du brome avec l'aldéhyde correspondant.

On connaît divers procédés de bromation des aldéhydes aromatiques. Ainsi on a proposé de conduire la bromation des hydroxy et/ou alcoxybenzaldéhydes dans divers milieux réactionnels. Le solvant le plus couramment utilisé est l'acide acétique glacial contenant éventuellement un acétate alcalin comme l'acétate de sodium, cf. DAKIN, Am. Chem. Journal 42 477-98 (1909); TORREY et al, J. Am. Chem. Soc. 31 583-585 (1909); O.S. BRADY et al, J. Chem. Soc. 107 1858-62 (1915); E. I. SHRINER et al, J. Am. Chem. Soc. 51 2194 (1929); R.A. Mc. IVOR et al, Cand. J. of Chem. 32 290-302 (1953); HENRY et al, J. Chem. Soc. (1930) 2279-89; F. MISANI et al, J. Org. Chem. 10 356 (1945); R. PSCHORR Ann. 391 23-39 (1912); brevet français 1 592 518. Bien que ce procédé conduise à d'excellents rendements en bromobenzaldéhydes, notamment dans le cas de la vanilline, il souffre de divers inconvénients qui le rende peu attrayant au plan industriel. En particulier ce procédé conduit en fin de réaction à une solution d'acide bromhydrique dans l'acide acétique à partir de laquelle il est difficile, voire pratiquement impossible, de récupérer HBr.

On a encore proposé (cf. R. PSCHORR loc. cit.) de remplacer l'acide acétique glacial par le chloroforme.

Dans le brevet français 72/38.410, publié sous le No. 2 177 693, on a décrit un procédé de bromation de la vanilline consistant à ajouter une solution de vanilline dans l'acide bromhydrique à 48% en poids d'HBr à du brome.

Les alcools inférieurs et notamment l'éthanol, ont été également utilisés comme milieu de bromation (cf. F. TIEMANN et al, Ber 7 615 [1874]). La formation conjointe de bromure de méthyle irrécupérable ou de bromure d'éthyle qui peut être difficile à valoriser dans le cadre d'une production importante de bromovanilline rend ce procédé peu attractif.

Dans tous les cas la réaction aboutit à la formation d'une molécule d'acide bromhydrique par molécule de bromobenzaldéhydes produit conformément au schéma réactionnel suivant:

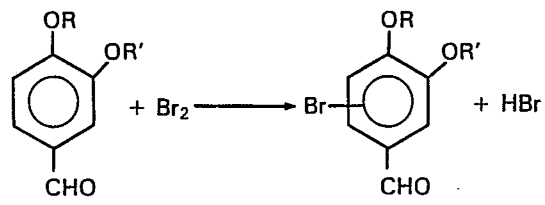

On constate que dans un tel processus seulement la moitié du brome engagé est utilisée pour la formation des bromobenzaldéhydes, l'autre moitié engendrant de l'acide bromhydrique où, selon le solvant utilisé, des bromures d'alkyle. La récupération et/ou la valorisation de ces sous-produits diminuent l'intérêt industriel de ce procédé quel que soit son mode de mise en œuvre.

Ainsi il ressort de cette analyse de l'état de la technique que la formation conjointe d'HBr résultant de l'emploi du brome comme agent de bromation pose un problème pour la mise en œuvre industrielle des procédés connus. La présente invention se propose précisément de résoudre ce problème en faisant appel à un procédé de bromation n'entraînant pas la formation conjointe d'acide bromhydrique.

Pour résoudre ce problème, le brevet US 3 303 224 a proposé de bromer des benzènes substitués à l'aide d'une solution d'acide bromique ou de bromate inorganique et d'un acide fort. Mais l'acide bromique est un composé instable et toxique, difficile à manipuler.

La demande de brevet FR-A 2 274 586 décrit l'utilisation d'un mélange acide bromhydrique/peroxyde d'hydrogène pour bromer des phénols tels que le bis(hydroxy-4 phényl)-2,2 propane. Cependant ce document ne suggère pas que ce système bromant puisse être utilisé pour bromer des composés aromatiques comportant une fonction aldéhyde, tels que ceux mis en œuvre dans la présente invention.

Plus particulièrement la présente invention a pour objet un procédé de préparation de brombenzaldéhydes substitués de formule générale:

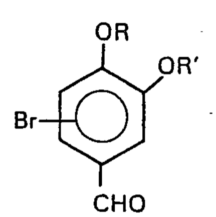

(I)

dans laquelle R et R' représentent un atome d'hydrogène ou un radical méthyle ou éthyle, caractérisé en ce que l'on fait réagir un benzaldéhyde substitué de formule:

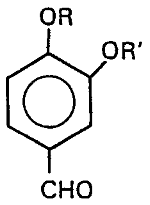

(II)

dans laquelle R et R' ont la signification donnée ci-avant avec un agent de bromation consistant en le couple formé par l'acide bromhydrique et un oxydant des ions bromures.

Parmi les aldéhydes de formule (I) qui peuvent être bromés par le procédé selon la présente invention on peut citer l'aldéhyde protocatéchique (dihydroxy--3,4 benzaldéhyde), la vanilline, l'isovanilline (hydroxy-3 méthoxy-4 benzaldéhyde), l'éthylvanilline, l'aldéhyde vératrique (diméthoxy-3,4 benzaldéhyde).

L'aldéhyde protocatéchique, la vanilline et l'éthylvanilline conduisent aux bromobenzaldéhydes correspondants comportant un brome en position méta par rapport au groupe aldéhyde et l'aldéhyde vératrique au bromo-2 hydroxy-4 méthoxy-3 benzaldéhyde.

Parmi les oxydants des ions bromures en brome convenant à la mise en œuvre du procédé selon l'invention on peut citer notamment l'eau oxygénée, l'acide nitrique et l'ion hypochlorite OCL$^-$ mise en œuvre de préférence sous forme d'hypochlorites alcalins tels que NaOCl et KOCl. L'eau oxygénée constitue l'oxydant préféré des ions bromure; dans ce cas la réaction peut être représentée par le schema suivant:

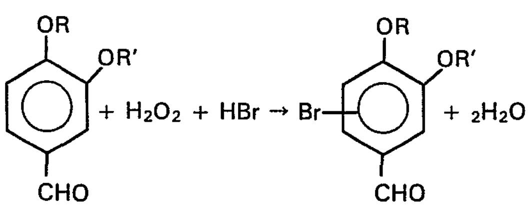

(a)

Bien qu'il soit, en général, connu d'oxyder les ions bromures en brome à l'aide de certains oxydants l'emploi du couple HBr/oxydant pour réaliser la bromation des hydroxy et/ou alcoxy benzaldéhydes pouvaient laisser craindre le déroulement de réactions d'oxydation et/or de substitution du produit de départ. Ainsi l'art antérieur enseigne l'oxydation par l'eau oxygénée de groupe aldéhyde selon une réaction du type Baeyer et Williger (cf. C.H. HASSAL, Organic Reactions, volume 9 pages 73 à 106 [1957]; J.E. LEFFLER, Chem. Rev. 45 pages 385 à 410 [1949]. La solution du problème posé par la bromation des aldéhydes aromatiques hydroxy et/ou alkoxy substitués ne s'imposait donc pas de façon évidente.

La quantité d'acide bromhydrique mise en œuvre dans le procédé selon la présente invention peut varier dans de larges limites. En générale la quantité d'acide bromhydrique exprimée en mole par mole de benzaldéhyde à bromer peut être de préférence au moins égale à la quantité stoechiométrique c'est-à-dire voisine d'une mole d'HBr par mole de benzaldéhyde. On pourrait, sans sortir du cadre de la présente invention, avoir recours à une quantité inférieure à la stochiométrie, mais cela se traduirait par un taux de transformation incomplet du benzaldéhyde. Au delà de cette quantité minimale, il n'y a pas de limite supérieure critique de la quantité d'acide bromhydrique. En fait la quantité d'acide bromhydrique mise en œuvre dépend du choix du milieu de réaction. C'est ainsi que si la réaction est conduite dans l'eau ou un composé organique, il n'est pas indispensable de faire appel à un excès d'acide bromhydrique; dans ce cas la quantité d'HBr peut représenter de 0,8 à 2 moles d'HBr par mole d'aldéhyde et de préférence de 1 à 1,5 mole HBr. On peut cependant choisir de conduire la réaction dans une solution aqueuse concentrée d'acide bromhydrique jouant le rôle de solvant du benzaldéhyde de départ.

La concentration de l'acide bromhydrique mis en œuvre n'est pas critique. Elle dépend essentiellement de considérations pratiques. Lorsque l'acide bromhydrique n'est pas utilisé comme solvant, l'utilisation d'acide dilué contribuerait à augmenter inutilement le volume réactionnel et par conséquent à diminuer la productivité de la réaction. Lorsque l'acide bromhydrique est utilisé comme milieu réactionnel, il est préférable que sa concentration soit suffisante pour obtenir la solubilité la plus élevée possible du benzaldéhyde de départ de façon à éviter soit de conduire la réaction sur une suspension de benzaldéhyde, soit de recourir à la présence d'un tiers solvant. Dans ce cas on fait appel de préférence aux solutions aqueuses d'HBr contenant de 40 à 55% en poids d'HBr et de préférence de 45 à 50% aisément disponibles dans le commerce. On pourrait cependant, sans sortir du cadre de la présente invention, utiliser des solutions aqueuses diluées d'acide bromhydrique comme milieu de réaction; la réaction se déroulerait alors en milieu hétérogène en raison de la faible solubilité des benzaldéhydes de départ dans l'acide bromhydrique dilué. Comme les bromobenzaldéhydes sont également insolubles dans ce milieu, il se forme au fur et à mesure du déroulement de la réaction un mélange solide de benzaldéhyde non bromé et de bromobenzaldéhyde.

La quantité d'agent oxydant des ions bromurés exprimée en mole par mole d'aldéhyde dépend évidemment de la nature de l'agent considéré. Ainsi lorsque l'agent oxydant est l'eau oxygénée cette quantité est de préférence voisine de la stochiométrie de la réaction représentée par le schéma (a) c'est-à-dire voisine de 1 mole par mole d'aldéhyde. Bien que l'on puisse sans inconvénient utiliser jusqu'à deux moles de $H_2O_2$ par mole d'aldéhyde celà ne se traduit par aucun avantage particulier. En pratique la quantité d'$H_2O_2$ est avantageusement comprise entre 0,8 et 1,5 mole par mole d'aldéhyde. L'emploi d'un défaut trop important d'eau oxygénée se traduirait par une limitation du taux de transformatiom de l'aldéhyde. Quand l'acide bromhydrique n'est pas

utilisé comme milieu mais comme simple vecteur de brome, il est préférable qu'il y ait un excès d'HBr par rapport à l'eau oxygénée; cet excès peut être de l'ordre de 0,01 à 0,5 mole d'HBr par mole d'eau oxygénée mise en œuvre. Lorsque l'acide nitrique est utilisé comme oxydant la réaction peut être représentée globalement par le schéma suivant:

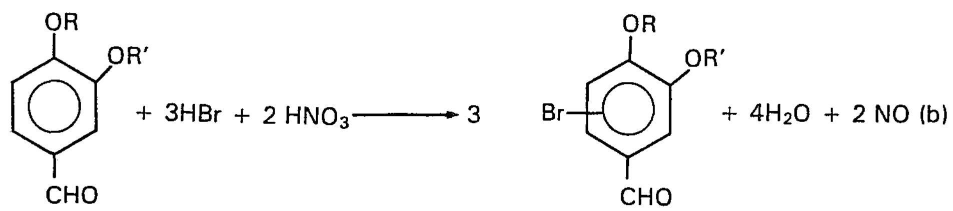


Dans ce cas la quantité d'acide nitrique exprimée en moles d'$HNO_3$ par mole de benzaldéhyde peut être voisine de la stoechiométrie de la réaction c'est-à-dire de 2/3 de mole par mole de benzaldéhyde ou s'en écarter quelque peu. En pratique la quantité d'acide nitrique peut varier de 0,35 à 1 mole par mole de benzaldéhyde et de préférence de 0,6 à 0,8 mole par mole d'HBr.

La concentration de l'acide nitrique n'est pas critique et dépend de considérations pratiques relatives à la productivité du procédé et à la disponibilité commerciale des solutions aqueuses d'$HNO_3$. En général on fait appel à des solutions aqueuses contenant de 20 à 75% en poids d'$HNO_3$.

On a constaté qu'il est préférable d'utiliser conjointement à l'acide nitrique de l'acide nitreux qui favorise un démarrage rapide de la réaction. Dans ce cas il est pratique de recourir à la présence d'un nitrite alcalin (nitrite de sodium ou de potassium) pour initier la réaction. Une quantité de l'ordre de 0,01 mole de nitrite par mole d'aldéhyde est en général suffisante pour initier la réaction; il n'est pas nécessaire qu'elle soit supérieure à 0,2 mole par mole d'aldéhyde. Une quantité de 0,05 à 0,15 mole est satisfaisante.

Quand on fait appel à l'ion hypochlorite comme oxydant, on l'utilise sous forme d'une solution aqueuse d'un hypochlorite alcalin tel que les hypochlorites de sodium et de potassium. En pratique on a recours aux solutions aqueuses d'hypochlorite de sodium dont la concentration n'est pas critique mais choisie en fonction des mêmes considérations pratiques que celles évoquées pour les autres oxydants. La quantité d'hypochlorite exprimée en mole par mole de benzaldéhyde est voisine de la stoechiométrie de la réaction représentée par le schéma réactionnel:

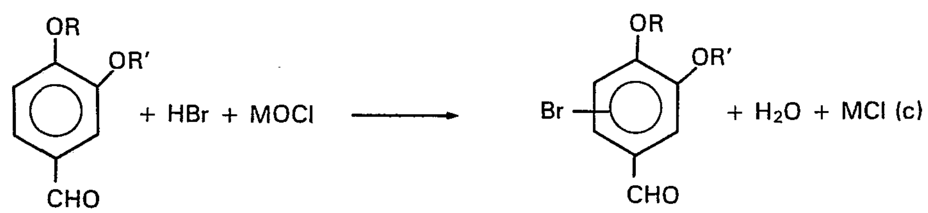


dans lequel M représente un métal alcalin; c'est-à-dire voisine de 1 mole par mole de benzaldéhyde. En général une quantité d'hypochlorite comprise entre 0,8 et 1,5 mole par mole de benzaldéhyde convient bien, encore qu'il soit possible d'opérer hors de ces limites sans sortir du cadre de l'invention. Ici encore il est préférable de conduire la réaction en présence d'un excès d'acide bromohydrique par rapport à la quantité stoechiométrique d'hypochlorite lorsque l'acide bromohydrique n'est pas utilisé comme milieu réactionnel. Cet excès peut être de l'ordre de 0,01 mole à 0,5 mole par rapport à la quantité stoechiométriquement nécessaire.

Lorsqu'on utilise un tiers composé organique comme milieu réactionnel on peut faire appel à tout composé inerte vis-à-vis du couple acide bromohydrique/oxydant ou de ses composants. On fait appel de préférence à des solvants des benzaldéhydes. Les hydrocarbures aliphatiques halogénés inférieurs (comportant de 1 à 4 atomes de carbone), les acides aliphatiques et les éthers aliphatiques inférieurs conviennent particulièrement bien; on peut citer notamment le choloforme, l'acide acétique anhydre, l'acide propionique anhydre, l'oxyde d'isopropyle.

On ne sortirait naturellement pas du cadre de l'invention en associant deux ou plusieurs solvants organiques ou un solvant organique et une solution aqueuse d'acide bromhydrique comme milieu réactionnel. Lorsqu'on utilise un acide carboxylique comme solvant, l'acide bromhydrique nécessaire au déroulement de la réaction peut être engendré «in situ» en chargeant un bromure alcalin (bromure de sodium par exemple).

La concentration du milieu en aldéhyde aromatique n'est pas critique et peut varier dans de larges limites. Son choix dépend de condidérations pratiques familières à l'homme de métier. Il doit réaliser le meilleur compromis possible entre

l'agitabilité du milieu et la productivité de l'appareillage.

La température à laquelle est conduite la réaction peut varier dans de larges limites. En général une température de 0 à 100°C convient bien. On opère de préférence dans un intervalle de température de 5 à 60°C.

Un mode préférée de réalisation de l'invention consiste à dissoudre l'aldéhyde aromatique à bromer dans une solution aqueuse d'acide bromhydrique de concentration comprise entre 40 et 55% en poids (de préférence entre 45 et 50% en poids) puis de mettre en contact cette solution avec une solution d'eau oxygénée à la température adéquate. Le bromobenzaldéhyde résultant de la réaction précipite au fur et à mesure de sa formation. En fin de réaction il suffit de l'isoler par filtration et de le laver à l'acide bromhydrique. Le filtrat et l'acide de lavage contenant le cas échéant l'aldéhyde non transformé sont réutilisés pour une nouvelle opération de bromation. Un tel procédé convient tout spécialement bien à la préparation des bromobenzaldéhydes de formule générale:

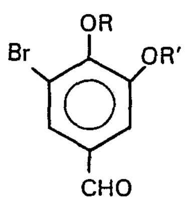

(III)

dans laquelle R' a la signification donnée ci-avant et notamment à la préparation de la bromo-5 vanilline.

Le procédé selon l'invention se prête particulièrement bien à une mise en œuvre continue.

Les exemples suivantes illustrent l'invention et montrent comment elle peut être mise en pratique.

*Exemple 1*

Dans un ballon en verre de 500 ml équipé d'un système d'agitation, d'un thermomètre, d'une ampoule de coulée et refroidi par un bain d'eau froide, on dissout à température ambiante 15,2 g de vanilline (0,1 mole) dans 180 ml de HBr à 48% en poids. On refroidit le mélange vers 5°C environ. On coule ensuite goutte à goutte 11,4 g d'$H_2O_2$ à 30% en poids (0,1 mole). La solution qui se colore en rouge, à l'endroit où tombe la goutte de $H_2O_2$, se décolore en quelques secondes. La masse réactionnelle devient très vite hétérogène. Lorsque la coulée est terminée on maintient 6 heures sous agitation à 5°C après quoi on ajoute 150 ml d'eau glacée à la masse réactionnelle. On filtre le précipité qui est lavé sur filtre par de l'eau glacée.

Après séchage sous vide vers 60°C, on obtient un précipité sec ayant un poids de 21,28 g et un point de fusion de 160°C. Le titre déterminé par chromatographie liquide sous haute pression est de 95% en bromo-5 vanilline et de 3% en vanilline.

Les résultats obtenus sont les suivants:

| | |
|---|---|
| Taux de transformation de la vanilline: | 96,2% |
| Rendement en bromo-5 vanilline/vanilline transformée: | 91,1% |
| Rendement en bromo-5 vanilline/vanilline engagée: | 87,6% |

*Exemple 2*

On opère comme à l'exemple 1 mais en utilisant 70 ml d'acide bromhydrique au lieu de 180 ml. Le milieu est hétérogène, la vanilline n'étant pas totalement dissoute. On obtient les mêmes résultats qu'à l'exemple 1.

*Exemple 3*

On charge à température ambiante 15,2 g de vanilline dans 100 ml d'acide bromhydrique à 47% en poids. Dans la suspension ainsi obtenue on ajoute goutte à goutte sous agitation 0,13 mole d'eau oxygénée sous forme d'une solution à 30% en poids (soit 14,73 g). La température s'élève progressivement à 30°C.

Lorsque l'addition est achevée on maintient le contenu du ballon 10 minutes à 30°C puis on traite la masse réactionnelle comme à l'exemple 1. Le filtrat est extrait trois fois par 100 ml de chloroforme à chaque fois. L'extrait chloroformique est évaporé à sec et le résidu mélangé au solide recueilli par filtration. L'ensemble est séché à poids constant puis la vanilline et la bromovanilline sont dosées comme à l'exemple 1.

Les résultats obtenus sont les suivants:

| | |
|---|---|
| Taux de transformation de la vanilline: | 86,1% |
| Rendement en bromo-5 vanilline/vanilline transformée: | 96,7% |

*Exemple 4*

On opère comme à l'exemple 3 en remplaçant l'acide bromhydrique à 47% en poids par 180 ml d'acide bromhydrique 3 N et en utilisant 0,11 mole d'eau oxygénée.

Les resultats obtenus sont les suivants:

| | |
|---|---|
| Taux de transformation de la vanilline: | 86,1% |
| Rendement en bromo-5 vanilline/vanilline transformée: | 96,7% |

*Exemple 5*

On opère comme à l'exemple 4 mais en remplaçant l'acide bromhydrique à 47% en poids par 50 ml d'acide bromhydrique 2N et 100 ml de chloroforme et l'eau oxygénée par 0,0187 mole d'acide nitrique (1,81 g d'une solution aqueuse à 65% en poids) et 0,01 mole de nitrite de sodium.

Les résultats obtenus sont les suivants:

| | |
|---|---|
| Taux de transformation de la vanilline: | 40,3% |
| Rendement en bromo-5 vanilline/vanilline transformée: | 58 % |

**Revendications**

1. Procédé de préparation de bromobenzaldéhydes substitués de formule générale:

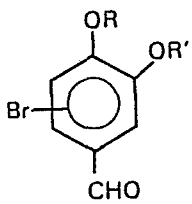

(I)

dans laquelle R et R' représentent un atome d'hydrogène ou un radical méthyle ou éthyle, caractérisé en ce que l'on fait réagir un benzaldéhyde substitué de formule:

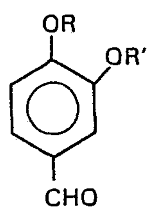

(II)

dans laquelle R et R' ont la signification donnée ci-avant avec un agent de bromation consistant en le couple formé par l'acide bromhydrique et un oxydant des ions bromures.

2. Procédé selon la revendication 1, caractérisé en ce que l'agent oxydant est pris dans le groupe formé par l'eau oxygénée, l'acide nitrique et les hypochlorites alcalins.

3. Procédé selon l'une quelconques des revendications 1 à 2, caractérisé en ce que la quantité d'acide bromhydrique est d'au moins 0,8 mole par mole de benzaldéhyde.

4. Procédé selon l'une quelconque des revendications 1 à 3, caractérisé en ce que la quantité d'eau oxygénée ou d'hypochlorite alcalin représente de 0,8 à 1,5 mole par mole de benzaldéhyde.

5. Procédé selon l'une quelconque des revendications 1 à 3, caractérisé en ce que la quantité d'acide nitrique représente de 0,35 à 1 mole de $HNO_3$ par mole de benzaldéhyde.

6. Procédé selon la revendication 5, caractérisé en ce que l'on opère en présence de 0,01 à 0,2 mole d'acide nitreux par mole de benzaldéhyde.

7. Procédé selon l'une quelconque des revendications 1 à 6, caractérisé en ce que l'on conduit la réaction dans une solution concentrée d'acide bromhydrique.

8. Procédé selon la revendication 7, caractérisé en ce que l'acide bromhydrique a une concentration de 45 à 60% en poids.

9. Procédé selon l'une quelconque des revendications 1 à 6, caractérisé en ce que l'on conduit la réaction dans un solvant organique du benzaldéhyde.

10. Procédé selon la revendication 9, caractérisé en ce que la réaction est conduite dans un solvant pris dans le groupe formé par les acides alcanoïques inférieurs, les hydrocarbures aliphatiques halogénés et les étheroxydes aliphatiques inférieurs.

11. Procédé selon l'une quelconques des revendications 1 à 10, caractérisé en ce que la réaction est conduite à une température de 0 à 100°C.

**Patentansprüche**

1. Verfahren zur Herstellung von substituierten Brombenzaldehyden der allgemeinen Formel

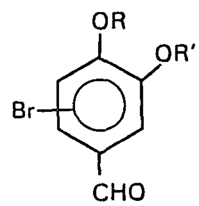

(I)

worin R und R' ein Wasserstoffatom oder einen Methyl- oder Ethylrest bedeuten, dadurch gekennzeichnet, dass man einen substituierten Benzaldehyd der Formel

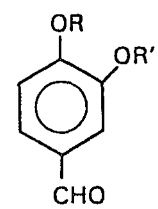

(II)

worin R und R' die vorstehend angegebene Bedeutung haben, mit einem Bromierungsmittel umsetzt, das aus dem Paar, gebildet von Bromwasserstoffsäure und einem Oxidationsmittel der Bromidionen, besteht.

2. Verfahren gemäss Anspruch 1, dadurch gekennzeichnet, dass das Oxidationsmittel aus der Gruppe, gebildet durch Wasserstoffperoxid, Salpetersäure und den Alkalihypochloriten, ausgewählt ist.

3. Verfahren gemäss einem der Ansprüche 1 bis 2, dadurch gekennzeichnet, dass die Menge an Bromwasserstoffsäure mindestens 0,8 Mol/Mol Benzaldehyd beträgt.

4. Verfahren gemäss einem der Ansprüche 1 bis 3, dadurch gekennzeichnet, dass die Menge an Wasserstoffperoxid oder Alkalihypochlorit 0,8 bis 1, Mol/Mol Benzaldehyd beträgt.

5. Verfahren gemäss einem der Ansprüche 1 bis 3, dadurch gekennzeichnet, dass die Menge an Salpetersäure 0,35 bis 1 Mol $HNO_3$/Mol Benzaldehyd beträgt.

6. Verfahren gemäss Anspruch 5, dadurch gekennzeichnet, dass man in Anwesenheit von 0,01 bis 0,2 Mol salpetriger Säure/Mol Benzaldehyd arbeitet.

7. Verfahren gemäss einem der Ansprüche 1 bis 6, dadurch gekennzeichnet, dass man die Reaktion in einer konzentrierten Bromwasserstoffsäurelösung durchführt.

8. Verfahren gemäss Anspruch 7, dadurch gekennzeichnet, dass die Bromwasserstoffsäure eine Konzentration von 45 bis 60 Gew.-% hat.

9. Verfahren gemäss einem der Ansprüche 1 bis 6, dadurch gekennzeichnet, dass man die Reaktion in einem organischen Lösungsmittel für Benzaldehyd durchführt.

10. Verfahren gemäss Anspruch 9, dadurch gekennzeichnet, dass die Reaktion in einem Lösungsmittel, genommen aus der Gruppe, die durch die niedrigen Alkansäuren, die halogenierten, aliphatischen Kohlenwasserstoffe und die niedrigen aliphatischen Ether gebildet ist, durchgeführt wird.

11. Verfahren gemäss einem der Ansprüche 1 bis 10, dadurch gekennzeichnet, dass die Reaktion bei einer Temperatur von 0 bis 100°C durchgeführt wird.

**Claims**

1. Process for preparing substituted bromobenzaldehydes of general formula:

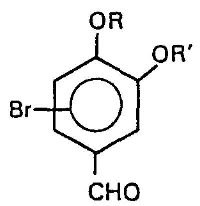

(I)

in which R and R' denote a hydrogen atom or a methyl or ethyl radical, in which a substituted benzaldehyde of formula:

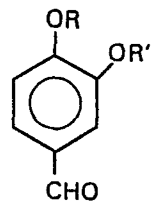

(II)

in which R and R' have the meaning given above is reacted with a brominating agent consisting of the pair formed by hydrobromic acid and an oxidizer of bromide ions.

2. Process according to claim 1, in which the oxidizing agent is taken from the group formed by hydrogen peroxide, nitric acid and alkali metal hypochlorites.

3. Process according to either of claims 1 to 2, in which the quantity of hxdrobromic acid is at least 0.8 mole per mole of the benzaldehyde.

4. Process according to any one of claims 1 to 3, in which the quantity of hydrogen peroxide or of alkali metal hypochlorite represents from 0.8 to 1.5 mole per mole of the benzaldehyde.

5. Process according to any one of claims 1 to 3, in which the quantity of nitric acid represents from 0.35 to 1 mole of $HNO_3$ per mole of the benzaldehyde.

6. Process according to claim 5, in which the operation is carried out in the presence of 0.01 to 0.2 mole of nitrous acid per mole of the benzaldehyde.

7. Process according to any one of claims 1 to 6, in which the reaction is conducted in a concentrated solution of hydrobromic acid.

8. Process according to claim 7, in which the hydrobromic acid is at a concentration of 45 to 60% by weight.

9. Process according to any one of claims 1 to 6, in which the reaction is conducted in an organic solvent for the benzaldehyde.

10. Process according to claim 9, in which the reaction is conducted in a solvent taken from the group formed by the lower alkanoic acids, halogenated aliphatic hydrocarbons and the lower aliphatic ethers.

11. Process according to any one of claims 1 to 10, in which the reaction is conducted at a temperature of 0 to 100°C.